# EUROPÄISCHE PATENTSCHRIFT

(11) **EP 2 983 609 B1**
(45) Veröffentlichungstag und Bekanntmachung des Hinweises auf die Patenterteilung: **16.10.2019**
(21) Anmeldenummer: 14720504.1
(22) Anmeldetag: 09.04.2014
(51) Int. Cl.: A61C 9/00, B05C 17/005, B65D 81/32, A61M 5/19, B05B 1/34, A61M 5/24, A61C 5/64, A61C 5/68

(54) **SPRITZE**
SYRINGE
SERINGUE

(30) Priorität: 09.04.2013 DE 102013103552
(43) Veröffentlichungstag der Anmeldung: 17.02.2016
(73) Patentinhaber: Ivoclar Vivadent AG, 9494 Schaan (LI)
(72) Erfinder: MÜLLER, Frank, A-6800 Feldkirch (AT); VOCKE, Lutz, CH-9400 Rohrschach (CH)
(74) Vertreter: Baronetzky, Klaus
(86) Internationale Anmeldenummer: PCT/EP2014/057167
(87) Internationale Veröffentlichungsnummer: WO 2014/167004

(56) Entgegenhaltungen:
- EP-A2- 1 825 927
- EP-A2- 1 825 927
- WO-A1-2007/131371
- WO-A1-2008/009143
- US-A- 4 538 920
- US-A- 4 969 747
- US-A- 4 969 747
- US-A1- 2010 318 063

## Beschreibung

Die Erfindung betrifft eine Spritze für das Ausbringen von fließfähigen dentaltechnischen Materialien, gemäß dem Oberbegriff von Anspruch 1.

Derartige Spritzen sind als sogenannte Doppelkammerspritzen seit langem bekannt. Eine Doppelkammerspritze ist beispielsweise aus der CH 669 164 A1 bekannt. Bei einer derartigen Spritze werden zwei Kolben für Vorratskammern gemeinsam betätigt. Hierdurch wird das in den Vorratskammern aufgenommene Material über die Auslässe der Vorratskammern ausgedrückt. Über Mischkanäle wird das Material einer Mischvorrichtung zugeleitet, die einen Ausgang aufweist, an den sich eine Kanüle anschließt. Eine solche Spritze ist auch aus der WO 2007/131371 A1 bekannt.

Derartige Doppelkammerspritzen haben sich auch für dentale Materialien seit langem bewährt, wenn es gilt, unterschiedliche Materialien kurz vor der Applikation zusammenzuführen und gemeinsam aufzubringen.

Gerade bei Verwendung von Dentalmaterialien bestehen jedoch bestimmte Randbedingungen und Gesichtspunkte, die bei der Konstruktion derartiger Spritzen beachtet werden müssen:
Dentalmaterialien sind häufig vergleichsweise hochwertig, so dass es ungünstig wäre, wenn Restmengen in der Spritze verbleiben, die nicht verwendbar sind. Daher ist man bemüht, Toträume zu vermeiden, und zwar sowohl in den Vorratskammern als auch - was in der Regel schwieriger zu bewerkstelligen ist - im Bereich des Ausbringens und des Mischens.

Zum Anderen ist gerade bei Dentalmaterialien eine gleichmäßige und gute Durchmischung relevant. Wenn die Mischungsverhältnisse nicht stimmen, ergeben sich gravierend andere Eigenschaften des Dentalmaterials, was zu Haftungsproblemen führen kann. Auch eine nicht-ausreichende Durchmischung kann entsprechende Degradationen nach sich ziehen.

Um diesem Problem zu begegnen, ist es vorgeschlagen worden, im Bereich der Kanüle eine Mischvorrichtung zu realisieren, beispielsweise durch das gezielte Einbauen von Strömungswiderständen, durch Gitter oder dergleichen.

Andererseits erhöhen derartige Strömungswiderstände den Ausbringwiderstand und werden als ungünstig empfunden. Zudem verlängert sich die Baulänge der Spritze, was als ergonomisch ungünstig empfunden wird. Als Beispiel hierfür sei auf die US 4 538 920 A1 verwiesen.

Daher hat man versucht, andere Lösungen für die Applikation von Substanzen, die aus zwei Komponenten bestehen, gerade im Dentalbereich zu schaffen. Als Beispiel hierfür sei auf die DE 101 24 819 A1 verwiesen.

Demgegenüber liegt der Erfindung die Aufgabe zugrunde, eine Spritze, insbesondere für das Ausbringen von fließfähigen dentaltechnischen Materialien, gemäß dem Oberbegriff von Anspruch 1 zu schaffen, bei der Toträume, in welchen sich unnutzbare Reste von Materialien ansammeln, nach Möglichkeit vermieden werden können, und ein geringer Ausbringwiderstand mit einer guten Durchmischung der Materialien verbindbar ist.

Diese Aufgabe wird erfindungsgemäß durch Anspruch 1 gelöst. Vorteilhafte Weiterbildungen ergeben sich aus den Unteransprüchen.

Erfindungsgemäß besonders günstig ist es, dass trotz der Verwendung von eher pastösen, aber noch fließfähigen Materialien, strömungstechnische Maßnahmen Wirkung zeigen, die bei geringem Strömungswiderstand eine gute Durchmischung erlauben. Überraschend zeigt es sich, dass trotz der Verwendung von viskosen Materialien gezielte Strömungsumlenkungen, wie sie an sich bekannt sind, bei relativ geringem Strömungswiderstand für die gute Durchmischung sorgen.

Besonders vorteilhaft ist die Realisierung von zwei Prallzonen, in welchen unterschiedliche Strömungen aufeinanderprallen, was eine überraschend eine bessere Durchmischung erzeugt als die Verwendung von Strömungswiderständen, wie sie beispielsweise aus der US 4 538 920 A1 bekannt sind.

Erfindungsgemäß ist es hierzu vorgesehen, die Mischvorrichtung mit einer Vormischzone und einer Mischzone zu versehen. In der Vormischzone wird ein Teilstrom des ersten Materials einem anderen Teilstrom eines weiteren Materials zugeleitet. Die Strömungsrichtungen eingangsseitig der Vormischzone, also die Strömungsrichtungen der Teilströme, sind einander zugewandt. Hierbei ist es günstig, wenn die Strömungsrichtungen nicht antiparallel zueinander verlaufen, sondern leicht schräg, beispielsweise in einem Winkel von 150° zueinander.

Gleichermaßen wird ein weiterer Teilstrom des ersten Materials einem Weiteren, also insgesamt dem vierten Teilstrom, zugeleitet, wobei der vierte Teilstrom aus dem zweiten Material besteht. Auch hier sind die Teilströme in ihren Strömungsrichtungen einander zugewandt, aber bevorzugt nicht antiparallel, sondern etwa im Winkel von etwa 150°. Es versteht sich, dass die exakte Ausrichtung in weiten Bereichen an die Erfordernisse anpassbar ist. Ein Winkel von 160°, einer 140°, oder aber auch 130° oder 120°, ist ebenso möglich.

Die vorgemischten Teilströme der beiden Materialien werden dann von der Vormischzone zur Mischzone geleitet. In der Vormischzone erfolgt eine Strömungsumlenkung um den halben Winkel der Strömungsrichtungen der beiden Teilströme. Bei einem Winkel von 150° würden die Teilströme also gemeinsam in einem spitzen Winkel von 75° zurückgeführt und der Mischzone zugeleitet.

Die vorgemischten Teilströme strömen zur Mischzone hin antiparallel aufeinander zu; auch hier gilt, dass eine leichte Winkelabweichung von 180° nicht unerwünscht ist, beispielsweise auch in der dritten Dimension.

Spätestens in der Mischzone erfolgt nun die Zusammenführung durch Aufeinanderprallen der Teilmischströme und Ablenkung in die dritte Dimension, also im Wesentlichen um 90°.

Es hat sich als besonders günstig herausgestellt, wenn das Prallelement schräge oder abgerundete oder ausgerundete Flächen aufweist. Hierdurch wird ein möglichst geringer Strömungswiderstand bereitgestellt, und die Haupt-Durchmischung erfolgt durch Aufeinanderprallen der Teilströme, die zwischen Vormischzone und Mischzone aufeinander zu laufen und über gleichsam über das Prallelement hinweg den gemeinsamen Weg zum Ausgang der Mischzone finden.

Auch der Gestaltung des Ausgangs der Mischzone kommt erfindungsgemäß eine besonders günstige Bedeutung zu. Bevorzugt ist der Ausgang im Bereich einer zusätzlichen Umlenkzone ausgebildet, die Schrägflächen aufweist, die sich konsistent zu den Schrägfächen des Prallelements erstrecken und eine Gegenprallfläche umgeben, die mit ihrer normalen zum Prallelement hinweist.

Hierdurch wird zwar ein Strömungswiderstand erzeugt. Dieser liegt jedoch im Bereich einer Strömungsaufweitung, an der dementsprechend geringere Strömungsgeschwindigkeiten herrschen, was strömungstechnisch dementsprechend einen verminderten Strömungswiderstand bedeutet. Überraschend ergibt sich hierdurch dennoch eine besonders gute Nachvermischung, die durch das Zusammenwirken des beispielsweise kegelförmigen Prallelements mit den Gegenschrägflächen und der Gegenprallfläche der Umlenkzone bei gleichzeitiger Strömungsaufweitung und anschließend hieran Strömungsverengung im Kanülenkanal bewirkt wird.

Die Kombination der Vormischzone, der Mischzone und der Umlenkzone ergibt damit die erfindungsgemäß besonders günstige Durchmischung bei geringem Strömungswiderstand, wobei die Voraussetzung für die Bereitstellung der Vormischzonen die Aufteilung der Ausgangsmaterialien in unterschiedliche Teilströme ist.

Dementsprechend sind erfindungsgemäß ausgangsseitig jeder Vorratskammer zwei Mischkanäle vorgesehen, die voneinander je divergieren und schräg auf den je gegenüberliegenden anderen Mischkanal des anderen Materials zu laufen. Die Vormischzone ergibt sich beim Aufeinanderprallen der je einander entsprechenden Teilströme und weist an ihrer von der Mischzone beabstandeten Seite eine Ausrundung auf, die eine widerstandsarmes freies Zuströmen der Teilströme in den einander zugewandten Mischkanälen ermöglicht, und hierdurch für die erwünschte Vorverwirbelung sorgt.

Erfindungsgemäß wird dementsprechend bewusst auf eine Vorumlenkung der Teilmischströme in Richtung Mischzone verzichtet, um die erfindungsgemäß günstige Vorverwirbelung nicht zu gefährden.

Erfindungsgemäß besonders günstig ist es, wenn die Mischkanäle einen breiten und flachen Querschnitt aufweisen, beispielsweise mit einem Höhen/Breiten-Verhältnis von 1:2.

Alle vier Mischkanäle sind dann bevorzugt in einer Ebene geführt, die senkrecht zur Ausbringrichtung der Spritze, also der Kanülenkanalachse, verläuft, deren Ebenennormale also parallel zur Kanülenkanalachse ist. Bevorzugt sind sowohl die Vormischzone als auch die Mischkanäle, aber auch das erfindungsgemäße Prallelement als entsprechende Vertiefung beziehungsweise Erhöhungen in einem Einsatzkörper ausgebildet, dessen Mischvorrichtungs-Seite in Ausbringrichtung weist und dementsprechend der Kanüle zugewandt ist. Lediglich die Umlenkzone ist dann in der Kanüle selbst ausgebildet, die die Umlenkzone umgebend dann eine gegen die Ausströmrichtung weisende flache Ausbildung aufweist.

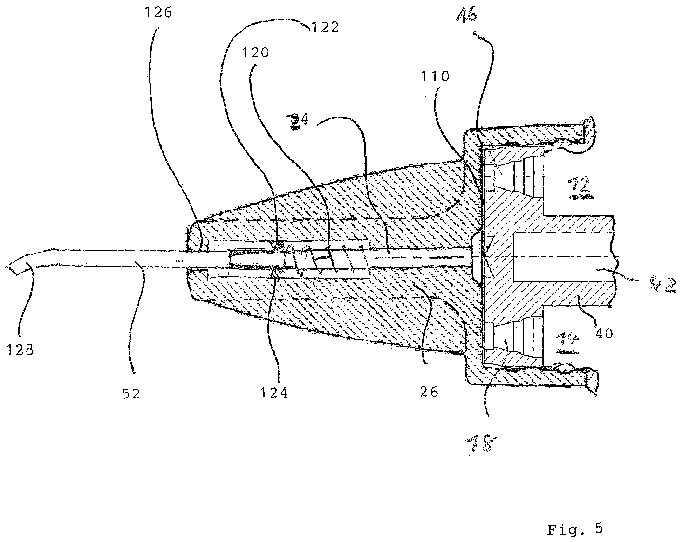

In erfindungsgemäß vorteilhafter Ausgestaltung lässt sich durch Wahl des Abstands zwischen der Kanüle und dem Einsatzkörper das Verhältnis zwischen Strömungswiderstand und Durchmischung nach Belieben einstellen; ein größerer Abstand, wie er aus Fig.5 ersichtlich ist, ergibt eine geringere Mischung und einen geringeren Strömungswiderstand.

Erfindungsgemäß besonders günstig ist es, wenn die Mischung durch eine vorgegebene Dosiervorrichtung realisiert ist, die eine vorgegebene Menge von Materialien sicher ausdrückt. Bei Durchströmung der Mischvorrichtung erfolgt bei Beginn des Abgabevorgangs und beim Ende des Abgabevorgangs die Abgabe mit einer typischerweise geringeren Strömungsgeschwindigkeit, denn die Masse wird bei Beginn der Dosierabgabe zunächst beschleunigt und gegen Ende abgebremst.

Erfindungsgemäß wirkt sich diese dosiertypische Tatsache jedoch nicht negativ auf die Durchmischung aus; die beim Durchtreten der Vormischzone zunächst langsamen Partikel der beiden Materialien werden beim Erreichen der Mischzone ausreichend beschleunigt, so dass letztlich dort die Durchmischung ausreichend stattfindet.

Dies gilt gleichermaßen beim Abbremsen gegen Ende des Dosiervorgangs; die Vermischung erfolgt dann bereits in ausreichendem Maße im Bereich der Vormischzone, so dass die geringere Strömungsgeschwindigkeit im Bereich der Mischzone sich ebenfalls nicht signifikant auf die Durchmischung auswirkt.

In besonders günstiger Ausgestaltung ist es vorgesehen, dass die Vorratskammern als Zylinder mit Kolben aufgebaut sind, wobei die Kolben gemeinsam über einen Kolbenantrieb betätigbar sind, und dass der Kolbenantrieb eine Dosiervorrichtung aufweist, welche bei Betätigung eines Betätigungselements eine je vorgegebene Menge aus den Vorratskammern abgibt.

Zudem lässt sich erfindungsgemäß in besonders günstiger Ausgestaltung die Dosierabgabe auch in den Bereich der Kanüle verlagern. In diesem Falle ist die Durchmischung - mit vergleichsweise hoher Strömungsgeschwindigkeit - im Bereich der Mischzone, der Vormischzone und der Umlenkzone bereits erfolgt, und die Dosierabgabe erfolgt dann lediglich für das bereits durchmischte Ausgangsmaterial durch Eindrücken eines Kanülenröhrchens, einer sogenannten Schubspitze, gegen die Federwirkung einer Druckfeder, die das Kanülenröhrchen gegen einen Anschlag aus dem Kanülenkanal nach vorne herausdrückt.

In diesem Zusammenhang besonders günstig ist die wahlweise Betätigungsmöglichkeit für die Applikation durch ein Betätigungselement, beispielsweise einen seitlich eindrückbaren Knopf, der auf Kolben der Vorratskammern wirkt, und andererseits über die Schubspitze.

In vorteilhafter Ausgestaltung ist es vorgesehen, dass die Mischvorrichtung im Bereich der Vormischzone eine Strömungsumlenkung aufweist, die insbesondere spitzwinklig ausgebildet ist.

In vorteilhafter Ausgestaltung ist es vorgesehen, dass zwei Vormischzonen - bezogen auf die Mischzone - einander gegenüberliegend angeordnet sind, und dass sich zwischen jeder Vormischzone und jedem Vorratskammer-Auslass mindestens ein Mischkanal erstreckt.

In vorteilhafter Ausgestaltung ist es vorgesehen, dass Vorratskammer-Auslässe einen kreisrunden Querschnitt und/oder die Mischkanäle einen rechteckigen Querschnitt und/oder das Prallelement einen pyramidenförmigen oder kegelförmigen oder kegelstumpfförmigen oder pyramidenstumpfförmigen Querschnitt aufweisen.

In vorteilhafter Ausgestaltung ist es vorgesehen, dass von jedem Auslass einer Vorratskammer mindestens zwei Mischkanäle ausgehen, die voneinander divergieren.

In vorteilhafter Ausgestaltung ist es vorgesehen, dass die Strömungsquerschnitte für die dentaltechnischen Materialien von der Vorratskammer ausgehend eine im wesentlichen konstante Querschnittsfläche bis zur Mischzone aufweisen und frei von Unstetigkeitsstellen wie Toträumen ausgebildet sind.

In vorteilhafter Ausgestaltung ist es vorgesehen, dass jeder Mischkanal zwischen dem Auslass und der Vormischzone, also im verzweigten Bereich, ein Längen/DurchmesserVerhältnis von mehr als 2:1, insbesondere zwischen 3:1 und 10:1, besonders bevorzugt zwischen 4:1 und 6:1, aufweist.

In vorteilhafter Ausgestaltung ist es vorgesehen, dass die Umlenkung in der Vormischzone in einer Ebene erfolgt, zu der die Umlenkung in der Mischzone eine dritte Dimension bildet.

In vorteilhafter Ausgestaltung ist es vorgesehen, dass die Strömungsrichtungen der dentaltechnischen Materialien zwischen den Vormischzonen aufeinander zu und zur Mischzone weisen.

In vorteilhafter Ausgestaltung ist es vorgesehen, dass an dem Ausgang der Mischzone eine Kanüle angeschlossen ist, die einen Kanülenkanal aufweist, an deren vorderer Spitze ein Kanülenröhrchen, insbesondere drehbar, lagert.

In vorteilhafter Ausgestaltung ist es vorgesehen, dass ein Kanülenröhrchen in einer Kanüle stromab eines Kanülenkanals, der sich an den Ausgang der Mischzone anschließt, schiebebeweglich gelagert ist, wobei das Kanülenröhrchen und der Kanülenkanal Durchmesser aufweisen, die sich voneinander um weniger als 30%, insbesondere um weniger als 10% unterscheiden.

In vorteilhafter Ausgestaltung ist es vorgesehen, dass das Kanülenröhrchen gegenüber dem Kanülenkanal schiebebeweglich und federbelastet gelagert ist, wobei die Federkraft weg von der Mischzone gerichtet ist und die Schiebebewegbarkeit des Kanülenröhrchens gegenüber der Kanüle durch Anschläge begrenzt ist.

In vorteilhafter Ausgestaltung ist es vorgesehen, dass die Spritze zwei Ausbringantriebe aufweist, die gleichzeitig und unabhängig voneinander betätigbar sind, nämlich ein Ausbringen über ein Eindrücken eines Kanülenröhrchen in eine Kanüle, und ein Ausbringen über eine Betätigung eines Betätigungselements, das auf einen gemeinsamen Kolbenantrieb für die Vorratskammern wirkt.

In vorteilhafter Ausgestaltung ist es vorgesehen, dass der Mischzonenausgang eine Umlenkzone aufweist, die sich zwischen dem Kanülenkanal und der Mischzone erstreckt und an welcher Umlenkzone Schrägflächen und Gegenprallflächen den Eingang des Kanülenkanals umgeben und auf das Prallelement zu weisen.

In vorteilhafter Ausgestaltung ist es vorgesehen, dass die Umlenkzone als kegelstumpfförmige Ausnehmung in der Kanüle ausgebildet ist, die sich über die Mischzone und das Prallelement erstreckt und einen größeren Strömungsquerschnitt als der Kanülenkanal aufweist.

In vorteilhafter Ausgestaltung ist es vorgesehen, dass sich Gegenprallflächen, die einen Kanülenkanal der Kanüle umgeben, mit ihrer Normalen parallel zur Achse des Kanülenkanals erstrecken.

In vorteilhafter Ausgestaltung ist es vorgesehen, dass die Mischvorrichtung eine Mischzone aufweist, die sich an eine Vormischzone anschließt, und dass die Mischzone ein Prallelement zur Umlenkung des vorgemischten Stroms von Dentalmaterialien zu einem Ausgang der Mischzone hin aufweist, und dass eine Dosiervorrichtung durch ein Betätigungselement für den gemeinsamen Kolbenantrieb der Kolben der Vorratskammer unabhängig betätigbar von einer Schubspitze (Kanülenröhrchen) ausgebildet ist, welche schiebebeweglich in oder an einer Kanüle gelagert ist, die sich an den Ausgang der Mischzone anschließt.

Weitere Vorteile, Einzelheiten und Merkmale ergeben sich aus der nachfolgenden Beschreibung mehrerer Ausführungsbeispiele der Erfindung anhand der Zeichnung.

Es zeigen:
- Fig.1: eine Schnittansicht eines Teiles einer erfindungsgemäßen Spritze, unter Darstellung der für die Erfindung wesentlichen Teile;
- Fig.2: eine Frontansicht auf einen Einsatzkörper zur Darstellung der Ausführungsform gemäß Fig. 1, unter Darstellung der Mischkanäle, der Vormischzone und der Mischzone;
- Fig.3: eine Schnittansicht des Einsatzkörpers, unter Darstellung des Prallelements;
- Fig.4: eine Ansicht der Teilströme der Mischkanäle, der Vormischzone und der Mischzone, zu der Ausführungsform gemäß Fig.1; und
- Fig.5: eine Schnittansicht einer modifizierten Ausführungsform des erfindungsgemäßen Spritze.

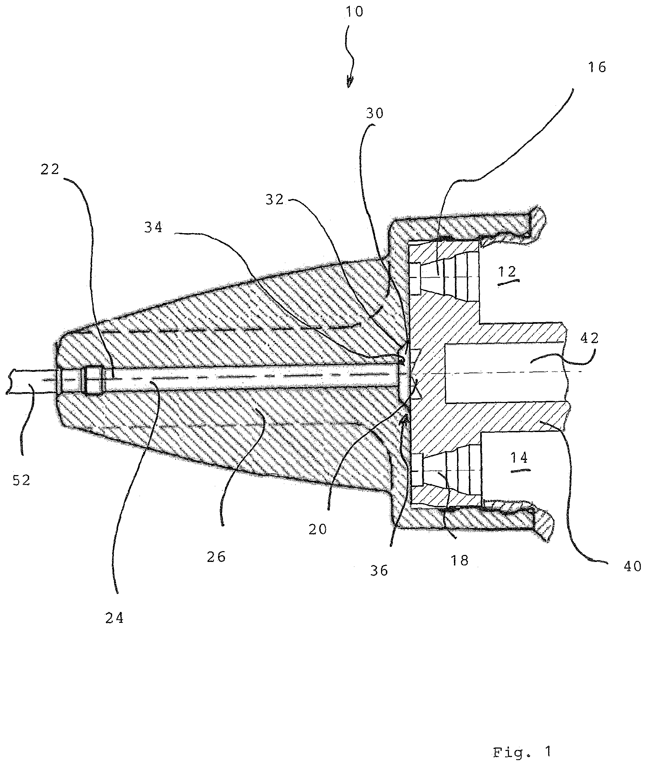

Die in Fig.1 dargestellte Spritze 10 weist Vorratskammern 12 und 14 auf, die aufgebrochen dargestellt sind und der Aufnahme von Dentalmaterialien dienen. Als derartige Dentalmaterialien können die folgenden Materialien in Mischungen verwendet werden:
Mischung aus Bisacrylamidderivat, Wasser, Alkohol, Bismethacrylamiddihydrogenphosphat, Acrylamidoaminosäure, hochdisperses Siliziumdioxid, Initiatoren, Katalysatoren und Kaliumfluorid.

Es versteht sich, dass diese Liste nicht als abschließende Liste zu verstehen ist und dass auch andere Materialien einsetzbar sind.

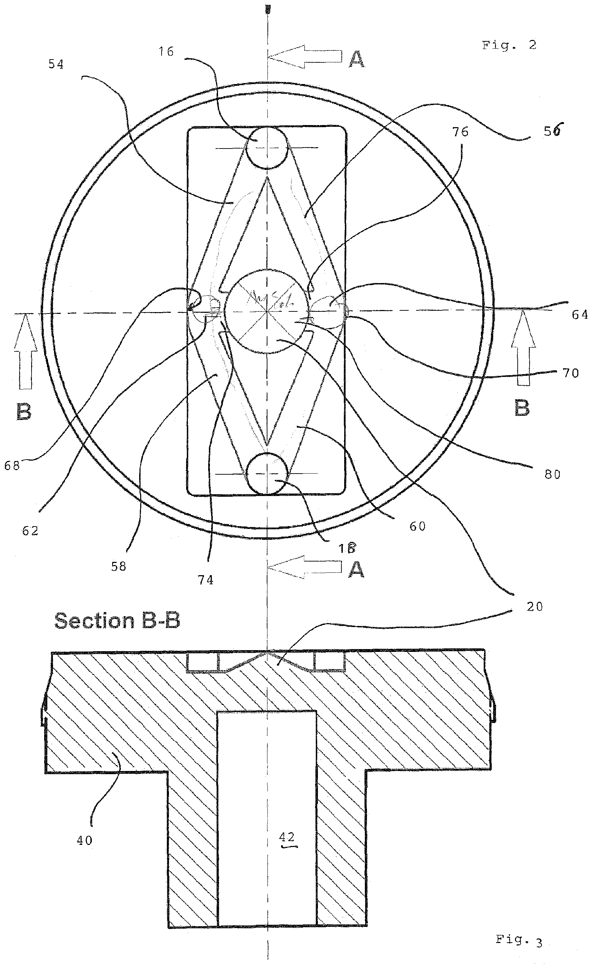

Ausgangsseitig der Vorratskammern sind Ausgänge 16 und 18 vorgesehen, die in Mischkanäle münden, deren Ausgestaltung anhand von Fig. 2 weiter unten erläutert wird.

Die Mischkanäle führen je Teilströme der Materialien aus Vorratskammern 12 und 14 aufeinander zu, und enden in Vormischzonen, die ebenfalls aus Fig. 2 ersichtlich sind. Von den Vormischzonen ausgehend werden Teilströme der Mischzone zugeleitet. Ein Teil der Mischzone wird von einem Prallelement 20 eingenommen, das aus Fig.1 ersichtlich ist. Hierdurch erfolgt eine Umlenkung der nun gemischten Materialien in Ausbringrichtung der Spritze, also in Richtung einer Achse 22 eines Kanülenkanals 24, der in einer Kanüle 26 ausgebildet ist.

Eingangsseitig des Kanülenkanals 24 ist eine Umlenkzone 30 ausgebildet, die dem Prallelement 20 gegenüber liegt. Die Umlenkzone 30 weist Schrägflächen 32 auf, die in ihrer grundsätzlichen Ausgestaltung das Prallelement 20 umgeben und gleichsinnig mit dessen Schrägflächen ausgebildet sind, sowie Gegenprallflächen 34, die sich zwischen den Schrägflächen 32 und dem Eingang des Kanülenkanals 24 erstrecken, und deren Flächen normale sich parallel zur Achse 22 erstreckt.

Die Mischkanäle, die Vormischzone, die Mischzone einschließlich des Prallelements 20 und Umlenkzone 30 bilden gemeinsam eine erfindungsgemäße Mischvorrichtung 36.

Die Kanüle 26 ist in an sich bekannter Weise auf die Spritze 10 im übrigen aufgesteckt oder aufgeschraubt. In dem dargestellten Ausführungsbeispiel liegt innen an ihr in Ausbringrichtung weisend ein Einsatzkörper 40 an, in dem der überwiegende Teil der Mischvorrichtung 36 ausgebildet ist.

Der Einsatzkörper 40 weist auch eine Führungsausnehmung 42 auf, in der ein Führungselement für den gemeinsamen Antrieb von Kolben der Vorratskammern 12 und 14 aufgenommen ist.

Bei Betätigen eines nicht-dargestellten Betätigungsknopfes, der sich seitlich der Spritze erstreckt, wird über sich im Winkel von 45° erstreckende und aufeinander zu gewandte Schubstangen ein Antriebselement betätigt, das an einer Zahnstange in Eingriff ist und über welches die Bewegung der Kolben realisiert wird.

Der Kanülenkanal 24 weist an seinem vorderen Ende 50 eine Ausnehmung für Aufnahme eines Kanülenröhrchens 52 auf. Das Kanülenröhrchen ist in beispielhafter Ausgestaltung auch aus Fig. 5 ersichtlich und bevorzugt drehbar in dem Kanülenkanal 24 gelagert. In der Ausführung gemäß Fig. 1 ist es drehbar in dem Kanülenkanal, aber nicht in Richtung der Achse 22 verschieblich, während es in der Ausführung gemäß Fig. 5 verschieblich gelagert ist. Es versteht sich, dass die Ausführungen der Figuren 1 und 5 - soweit sie sich unterscheiden -, je miteinander kombinierbar sind, also beispielsweise auch eine verschiebliche Lagerung bei der Ausführungsform gemäß Fig. 1 vorgesehen sein kann.

Aus Fig. 2 ist ersichtlich, in welcher Weise die Einzelteile der Mischvorrichtung 36 in dem Einsatzkörper 40 realisiert sein können. Anschließend an die Ausgänge 16 und 18 erstrecken sich je die Mischkanäle 54, 56, 58 und 60. Hierbei divergieren die Mischkanäle 54 und 56 einerseits und 58 und 60 andererseits je voneinander, beispielsweise in einem Winkel zwischen 20 und 70°, bevorzugt in einem Winkel zwischen 40 und 50°, beispielsweise in einem Winkel von 45°. Sie sind zueinander symmetrisch, so dass sämtliche Mischkanäle 54 bis 60 gemeinsam betrachtet eine Raute aufspannen.

Die Mischkanäle 54 bis 60 enden je in Vormischzonen. Hierbei enden die Mischkanäle 54 und 58 in der Vormischzone 62 und die Mischkanäle 56 und 60 in der Vormischzone 64. Jede Vormischzone weist nach außen hin, also an den einander gegenüberliegenden Seitenflächen je Ausrundungen 68 und 70 auf. Die Ausgänge 74 und 76 der Vormischzonen 62 und 64 sind einander zugewandt und münden in der Mischzone 80.

Die Mischzone 80 weist das Prallelement 20 als zentralen Körper auf. Das Prallelement 20 ist entweder nach der Art einer flachen Pyramide oder als Kegel, oder als gegebenenfalls als Kegelstumpf ausgebildet. Alternativ kann es kugelabschnittförmig oder paraboloid ausgebildet sein. Über das Prallelement 20 hinweg prallen die Teilströme aus den Ausgängen 74 und 76 der Vormischzonen 62 und 64 aufeinander und werden in der Mischzone 80 dementsprechend nachvermischt.

Wie aus Fig. 3 ersichtlich ist, hat das Prallelement 20 einen recht großen Spitzenwinkel. Der Spitzenwinkel ist jedenfalls ein stumpfer Winkel und beträgt beispielsweise zwischen 110 und 160°, bevorzugt etwa 135°.

Es ist ersichtlich, dass das Prallelement 20 vollständig in dem Einsatzkörper 40 ausgebildet ist.

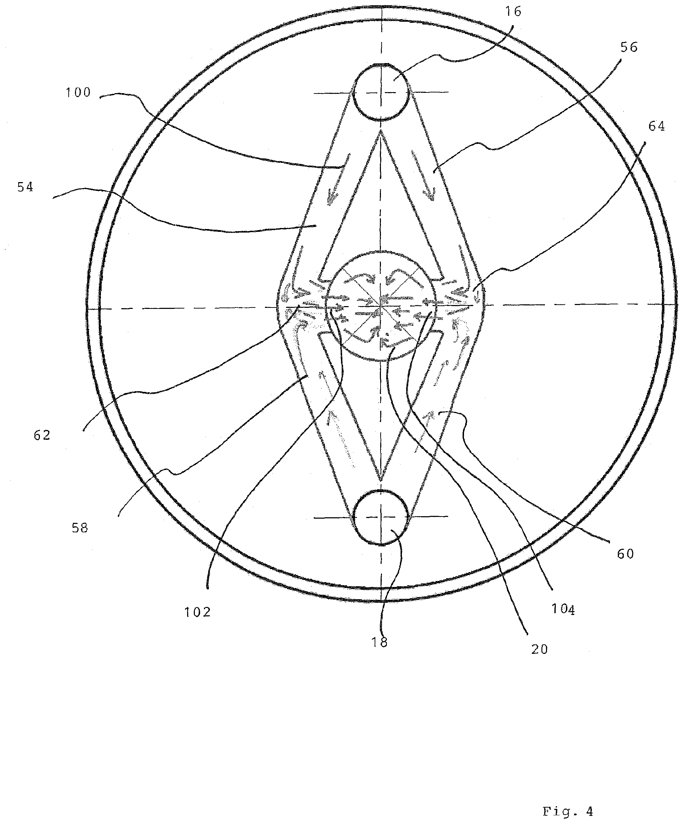

Aus Fig. 4 ist ersichtlich, wie die Teilströme 100 in den Mischkanälen 54 bis 60 von den Ausgängen 16 und 18 ausgehend zunächst divergieren und dann je in den Vormischzonen 62 und 64 aufeinander aufprallen und dort umgelenkt werden.

Über dem Prallelement 20 erfolgt eine weiteres Aufprallen der Teilströme 102 und 104 in die dritte Dimension, in Fig. 4 also aus der Zeichnungsebene heraus. Auch diese doppelte Umlenkung in zwei Dimensionen führt zu einer guten Durchmischung.

Fig. 5 zeigt eine modifizierte Ausgestaltung der erfindungsgemäßen Spritze. Im rückwärtigen Bereich der Kanüle ist ein Spalt 110 von z. B. 10µ Dicke zwischen der Kanüle 26 und dem Einsatzkörper 40, der temperaturbedingte Bewegungen der Einzelteile der Spritze auffängt. Durch den gemeinsamen Eingang an dem Einlass des Kanülenkanals 24 wird das erfindungsgemäß durchmischte Material gemeinsam ausgedrückt.

In dem vorderen Bereich des Kanülenkanals 24 ist das Kanülenröhrchen 52 schiebebeweglich und drehbeweglich geführt. Hierzu ist der Kanülenkanal 24 als Rohr ausgebildet, das in die Kanüle 26 eingesetzt ist. Auf diesem Rohr 24 ist dann das Kanülenröhrchen 52 geführt. Den Kanülenkanal 24 im vorderen Bereich umgeben ist eine Druckfeder 120 aufgenommen, die zwischen der Kanüle 26 und einer rückwärtigen Verdickung 122 des Kanülenröhrchens 52 vorgespannt ist. Die Bewegung des Kanülenröhrchens 52 nach vorne wird durch einen Anschlag 124 begrenzt, der gegen die Verdickung 122 wirkt. Zudem ist das Kanülenröhrchen 52 auslassseitig an einer recht engen Durchtrittsausnehmung 126 der Kanüle 26 geführt, so dass durch die beabstandete Führung eine gute seitliche Stabilisierung möglich ist.

Durch die Drehbarkeit des Kanülenröhrchens lässt sich besonders günstig die Spritze auch bei seitlicher Betätigung, beispielsweise über einen Betätigungsknopf, handhaben. Der Betätigungsknopf kann dann an der gleichen Stelle verbleiben, unabhängig davon, ob der Zahnarzt den Unterkiefer oder den Oberkiefer behandelt.

In vorteilhafter Ausgestaltung ist ein Kanülenrohr 24 fest in der Kanüle 26 befestigt, beispielswiese durch Kleben, Pressen, und Spritzen oder durch Schweißen. Die Kanüle wird bei dieser Ausgestaltung drehbar auf dem Kanülenröhrchen 52 aufgeprellt.

In weiterer modifizierter Ausgestaltung ist das Kanülenröhrchen aus einem biegsamen Material ausgebildet, das bevorzugt unelastisch und biegeschlaff ist, so dass es nach einer Biegung in eine bestimmte Richtung ohne Zurückzufedern an dieser Stelle verbleibt.

In dem dargestellten Ausführungsbeispiel ist das Kanülenröhrchen 52 endseitig mit einer Abkröpfung 128 versehen. Es versteht sich, dass die Abkröpfung auch stärker oder weniger stark als in Fig. 5 dargestellt ausgebildet sein kann. Bei einer geringen Abkröpfung ist es möglich, durch frontalen Druck auf das Kanülenröhrchen 52 Dentalmaterial nach der Art einer Schubspitze auszudrücken. Das Dentalmaterial ist in dem Zustand, in dem es sich im Kanülenröhrchen 52 befindet, bereits perfekt durchgemischt, so dass die geringe Strömungsgeschwindigkeit, die beim Beginn und beim Ende des Einschiebens des Kanülenröhrchens auftritt, keine nachteilige Wirkung auf die Durchmischung hat.

## Patentansprüche

1. Spritze, insbesondere für das Ausbringen von fließfähigen dentaltechnischen Materialien, mit einem Spritzengehäuse, mit mindestens zwei quer - zur Ausbringrichtung betrachtet - nebeneinanderliegenden Vorratskammern (12 und 14) für dentaltechnische Materialien, und mit einer Mischvorrichtung (36), die über zwei Mischkanäle (54, 56, 58 und 60) verfügt, die je an dem Auslass einer Vorratskammer (12 und 14) angeschlossen sind, **dadurch gekennzeichnet, dass** die Mischvorrichtung (36) eine Mischzone (80) aufweist, die sich an eine Vormischzone (64) anschließt und dass die Vormischzone (64) zwei Eingänge, je einen für jedes der zu mischenden Dentalmaterialien, und einen Ausgang aufweist, wobei die Eingänge aufeinander zu weisen und der Ausgang der Vormischzone (64) sich quer zu den Eingängen erstreckt, und dass der Eingang der Mischzone (80) sich an den Ausgang der Vormischzone (64) anschließt und die Mischzone (80) ein Prallelement (20) aufweist, welches dazu ausgebildet ist, den vorgemischten Strom von den Dentalmaterialien zu einem Ausgang der Mischzone (80) hin umzulenken.

2. Spritze nach Anspruch 1 , **dadurch gekennzeichnet, dass** die Mischvorrichtung (36) im Bereich der Vormischzone (64) eine Strömungsumlenkung aufweist, die insbesondere spitzwinklig ausgebildet ist.

3. Spritze nach einem der vorhergehenden Ansprüche, **dadurch gekennzeichnet, dass** zwei Vormischzonen (62, 64) - bezogen auf die Mischzone (80) - einander gegenüberliegend angeordnet sind, und dass sich zwischen jeder Vormischzone (62, 64) und jedem Vorratskammer-Auslass mindestens ein Mischkanal erstreckt.

4. Spritze nach einem der vorhergehenden Ansprüche, **dadurch gekennzeichnet, dass** die Vorratskammer-Auslässe einen kreisrunden Querschnitt und/oder die Mischkanäle (54, 56, 58 und 60) einen rechteckigen Querschnitt und/oder das Prallelement (20) einen pyramidenförmigen oder kegelförmigen oder kegelstumpfförmigen oder pyramidenstumpfförmigen Querschnitt aufweist.

5. Spritze nach einem der vorhergehenden Ansprüche, **dadurch gekennzeichnet, dass** von jedem Auslass einer Vorratskammer (12 und 14) mindestens zwei Mischkanäle (54, 56, 58 und 60) ausgehen, die voneinander divergieren.

6. Spritze nach einem der vorhergehenden Ansprüche, **dadurch gekennzeichnet, dass** die Strömungsquerschnitte für die dentaltechnischen Materialien von der Vorratskammer (12 und 14) ausgehend eine im wesentlichen konstante Querschnittsfläche bis zur Mischzone (80) aufweisen und frei von Unstetigkeitsstellen wie Toträumen ausgebildet sind.

7. Spritze nach einem der vorhergehenden Ansprüche, **dadurch gekennzeichnet, dass** jeder Mischkanal zwischen dem Auslass und der Vormischzone (64), also im verzweigten Bereich, ein Längen/Durchmesser- Verhältnis von mehr als 2:1, insbesondere zwischen 3:1 und 10:1, besonders bevorzugt zwischen 4:1 und 6:1, aufweist.

8. Spritze nach einem der Ansprüche 2 bis 7, **dadurch gekennzeichnet, dass** die Strömungsumlenkung in der Vormischzone (64) in einer Ebene erfolgt, zu der die Umlenkung in der Mischzone (80) eine dritte Dimension bildet.

9. Spritze nach einem der Ansprüche 3 bis 8, **dadurch gekennzeichnet, dass** die Strömungsrichtungen der dentaltechnischen Materialien zwischen den Vormischzonen (62, 64) aufeinander zu und zur Mischzone (80) weisen.

10. Spritze nach einem der vorhergehenden Ansprüche, **dadurch gekennzeichnet, dass** an dem Ausgang der Mischzone (80) eine Kanüle (26) angeschlossen ist, die einen Kanülenkanal (24) aufweist, an deren vorderer Spitze ein Kanülenröhrchen (52), insbesondere drehbar, lagert.

11. Spritze nach Anspruch 10, **dadurch gekennzeichnet, dass** der Mischzonenausgang eine Umlenkzone (30) aufweist, die sich zwischen dem Kanülenkanal (24) und der Mischzone (80) erstreckt und an welcher Umlenkzone (30) Schrägflächen und Gegenprallflächen den Eingang des Kanülenkanals (24) umgeben und auf das Prallelement (20) zu weisen.

12. Spritze nach Anspruch 11, **dadurch gekennzeichnet, dass** die Umlenkzone (30) als kegelstumpfförmige Ausnehmung in der Kanüle (26) ausgebildet ist, die sich über die Mischzone (80) und das Prallelement (20) erstreckt und einen größeren Strömungsquerschnitt als der Kanülenkanal (24) aufweist.

13. Spritze nach einem der Ansprüche 10 bis 12, **dadurch gekennzeichnet, dass** sich Gegenprallflächen, die den Kanülenkanal (24) der Kanüle (26) umgeben, mit ihrer Normalen parallel zur Achse (22) des Kanülenkanals (24) erstrecken.

## Claims

1. A syringe, especially for delivering flowable dental materials, comprising a syringe housing, at least two adjacent storage chambers (12 and 14) for dental materials, as viewed transversely to the delivery direction, and a mixing device (36) which has two mixing channels (54, 56, 58 and 60) which each are connected to the outlet of a storage chamber (12 and 14), **characterized in that** the mixing device (36) has a mixing zone (80) adjoining a premixing zone (64) and **in that** the premixing zone (64) comprises two inlets, one for each of the dental materials to be mixed, and one outlet, the inlets facing each other and the outlet of the premixing zone (64) extending transversally to the inlets, and **in that** the inlet of the mixing zone (80) adjoins the outlet of the premixing zone (64) and the mixing zone (80) comprises an impact element (20) adapted to deflect the premixed stream of the dental materials to an outlet of the mixing zone (80).

2. The syringe according to claim 1, **characterized in that** the mixing device (36) has a flow deflection in the region of the premixing zone (64), which flow deflection especially is configured acutely-angled.

3. The syringe according to one of the preceding claims, **characterized in that** two premixing zones (62, 64) - with respect to the premixing zone (80) - are arranged opposite to each other, and **in that** at least one mixing channel extends between each premixing zone (62, 64) and each storage chamber outlet.

4. The syringe according to one of the preceding claims, **characterized in that** the storage chamber outlets have a circular cross-section and/or the mixing channels (54, 56, 58 and 60) have a rectangular cross-section and/or the impact element (20) has a pyramidal or conical or frusto-conical or truncated pyramidal cross-section.

5. The syringe according to one of the preceding claims, **characterized in that** at least two mixing channels (54, 56, 58 and 60) extend from each outlet of a storage chamber (12 and 14) which divert from each other.

6. The syringe according to one of the preceding claims, **characterized in that** the flow cross-sections for the dental materials starting from the storage chamber (12 and 14) have a substantially constant cross-sectional area up to the mixing zone (80) and are formed free of discontinuities such as dead spaces.

7. The syringe according to one of the preceding claims, **characterized in that** each mixing channel has a length/diameter ratio of more than 2:1, especially between 3:1 and 10:1, particularly preferably between 4:1 and 6:1 between the outlet and the premixing zone (64), i.e. in the branched region.

8. The syringe according to one of claims 2 to 7, **characterized in that** the flow deflection occurs in the premixing zone (64) in a plane to which the deflection in the mixing zone (80) forms a third dimension.

9. The syringe according to one of claims 3 to 8, **characterized in that** the flow directions of the dental materials between the premixing zones (62, 64) points towards each another and towards the mixing zone (80).

10. The syringe according to one of the preceding claims, **characterized in that** a cannula (26) is connected to the outlet of the mixing zone (80) and has a cannula steering channel (24) at the front tip of which a cannula tube (52) is mounted, and especially is rotatably mounted.

11. The syringe according to Claim 10, **characterized in that** the mixing zone outlet has a deflection zone (30) which extends between the cannula channel (24) and the mixing zone (80) and at which deflection zone (30) oblique surfaces and counterimpact surfaces surround the inlet of the cannula channel (24) and point towards the impact element (20).

12. The syringe according to claim 11, **characterized in that** the deflection zone (30) is designed as a frusto-conical recess in the cannula (26) which extends across the mixing zone (80) and the impact element (20) and has a larger flow cross-section than the cannula channel (24).

13. The syringe according to one of claims 10 to 12, **characterized in that** counterimpact surfaces, which surround said one cannula channel (24) of the cannula (26), extend with their normal parallel to the axis (22) of the cannula channel (24).

## Revendications

1. Seringue, en particulier pour la distribution de matériaux dentaires fluides, avec un boîtier de seringue, avec au moins deux réservoirs de stockage (12 et 14) adjacents - vus dans de sens de la distribution - pour matériaux dentaires, et avec un dispositif de mélange (36), qui dispose de deux conduits mélangeurs (54, 56, 58 et 60), dont chacun est connecté à la sortie d'un réservoir de stockage (12 et 14), **caractérisé en ce que** le dispositif de mélange (36) présente une zone de mélange (80) qui est connecté à une zone de prémélange (64) et que la zone de pré-mélange (64) dispose de deux entrées, une pour chacun des matériaux dentaires à mélanger, et une sortie, où les entrées sont dirigées les unes vers les autres et la sortie de la zone de pré-mélange (64) s'étend transversalement aux entrées, et que l'entrée de la zone de mélange (80) se connecte à la sortie de la zone de prémélange (64) et la zone de mélange (80) dispose d'un élément d'impact (20) qui est conçu pour dévier le flux prémélangé des matériaux dentaires vers une sortie de la zone de mélange (80).

2. Seringue selon la revendication 1, **caractérisé en ce que** dans la zone de prémélange (64) le dispositif de mélange (36) présente une déviation de flux, qui forme notamment un angle aigu.

3. Seringue selon l'une des revendications précédentes, **caractérisée en ce que** deux zones de prémélange (62, 64) - par rapport à la zone de mélange (80) - sont disposées l'une en face de l'autre, et qu'entre chaque zone de prémélange (62, 64) et chaque point de sortie d 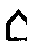 un réservoir de stockage s'étend au moins un conduit mélangeur.

4. Seringue selon l'une des revendications précédentes, **caractérisée en ce que** les sorties de réservoir présentent une section transversale circulaire et/ou les conduits mélangeurs (54, 56, 58 et 60) présentent une section rectangulaire et/ou l'élément d'impact (20) présente une section pyramidale ou conique ou tronconique.

5. La seringue selon l'une des revendications précédentes, **caractérisée en ce qu'**au moins deux conduits mélangeurs (54, 56, 58 et 60) qui divergent l'un de l'autre sortent de chaque point de sortie d'un réservoir de stockage (12 et 14).

6. La seringue selon l'une des revendications précédentes, **caractérisée en ce que** les sections transversales d'écoulement pour les matériaux dentaires de la chambre de stockage (12 et 14) ont une section transversale essentiellement constante jusqu'à la zone de mélange (80) et sont exempts de points de discontinuité tels que les zones mortes.

7. Seringue selon l'une des revendications précédentes, **caractérisée en ce que** chaque conduit mélangeur entre la sortie et la zone de prémélange (64), c'est-à-dire dans la plage ramifiée, un rapport longueur/diamètre de plus de 2:1, en particulier entre 3:1 et 10:1, de manière particulièrement préférée entre 4:1 et 6:1.

8. Seringue selon l'une des revendications 2 à 7, **caractérisée en ce que** la déviation de l'écoulement a lieu dans la zone de prémélange (64) dans un plan par rapport auquel la déviation dans la zone de mélange (80) forme une troisième dimension.

9. Seringue conformément à l'une quelconque des revendications 3 à 8, **caractérisée en ce que** les sens d'écoulement des matériaux dentaires entre les zones de prémélange (62, 64) sont dirigés les uns vers les autres et vers la zone de mélange (80).

10. Seringue selon l'une des revendications précédentes, **caractérisée en ce qu'**à la sortie de la zone de mélange (80) une canule (26) est connectée, qui présente un canal de canule (24), à l'extrémité avant duquel se trouve un tube de canule (52), en particulier de manière rotative.

11. Seringue selon la revendication 10 **caractérisée en ce que** la sortie de la zone de mélange a une zone de déviation (30) qui s'étend entre le canal de canule (24) et la zone de mélange (80) et à ladite zone de déviation (30) des surfaces inclinées et des surfaces de contre-impact entourent l'entrée du canal de la canule (24) et sont dirigées vers l'élément d'impact (20).

12. Seringue selon la revendication 11, **caractérisée en ce que** la zone de déviation (30) est formée comme une cavité tronconique dans la canule (26) qui s'étend sur la zone de mélange (80) et l'élément d'impact (20) et présente une plus grande section transversale d'écoulement que le canal de la canule (24).

13. La seringue selon l'une des revendications 10 à 12, **caractérisée en ce que** les surfaces de contre-impact qui entourent un canal de canule (24) de la canule (26) s'étendent avec leurs normales parallèles à l'axe (22) du canal de la canule (24).
